# FASCICULE DE BREVET EUROPEEN

(11) **EP 1 000 594 B1**
(45) Date de publication et mention de la délivrance du brevet: **03.03.2004**
(21) Numéro de dépôt: 98630067.1
(22) Date de dépôt: 13.11.1998
(51) Int. Cl.: A61F 2/36

(54) **Piton de fixation osseuse pour tête articulaire fémorale**
Befestigungsschaft für Femurkopf
Fixation stem for femoral head

(43) Date de publication de la demande: 17.05.2000
(73) Titulaire: Bone & Joint Research S.A., 3644 Kayl (LU)
(72) Inventeur: Vander Maren, Christian, 1340 Ottignies (BE); Thiery, Jacques, 1390 Grez-Doiceau (BE); Deneufbourg, Jean, 1300 Wavre (BE); Autrique, Jean-Claude, 1300 Wavre (BE); Geulette, Bernard, 1050 Bruxelles (BE); Boucquey, Pierre, 7700 Mouscron (BE); Ghosez, Jean-Pierre, 5100 Jambes (BE); Moulin, Jean, 3644 Kayl (LU); Thiltges Paul, B-1020 Bruxelles (BE); Escolar-Thiltges Laurence, B-1120 Bruxelles (BE)
(74) Mandataire: Schmitz, Jean-Marie

(56) Documents cités:
- DE-A- 2 537 807
- DE-A- 2 627 569
- FR-A- 2 578 738
- FR-A- 2 678 509
- US-A- 4 532 661
- US-A- 4 979 958

## Description

La présente invention concerne un piton de fixation osseuse pour tête articulaire fémorale selon la partie précaractérisante de la revendication 1. Ce piton est utilisé pour les opérations de prothèse de la hanche.

Dans les prothèses de la hanche on utilise un piton de fixation osseuse pour tête articulaire fémorale comprenant au moins une partie d'assemblage avec la tête articulaire ayant un effet de coincement dans une position variable selon le type de tête articulaire choisie et une partie permettant la fixation à l'os fémoral par l'intermédiaire d'un composé ou ciment introduit à l'état pâteux remplissant l'espace entre l'os et le piton de fixation.

La publication DE-OS 26 27 567 décrit un piton de fixation osseuse pour tête articulaire fémorale comprenant une partie d'assemblage avec cette tête articulaire et une partie de fixation destinée à être fixée à l'os fémoral, la partie d'assemblage et la partie de fixation étant reliées par un rebord.

La mise en place d'un piton de fixation scellé dans une cavité osseuse rencontre de nombreuses difficultés per-opératoires qui se traduisent par des descellements précoces accompagnés de douleurs et lésions osseuses nécessitant une ré-intervention chirurgicale.

Une de ces difficultés est due à la présence de gaz (air ou vapeur de monomère) dans le mélange ou dans le composé de remplissage à l'état pâteux qu'il faut éviter de piéger dans ce composé de remplissage. Une autre difficulté provient de la manoeuvre de remplissage de la cavité osseuse qui saigne et qui contient des débris de moelle graisseuse libérés lors de la préparation du logement.

Dans les deux cas le composé de remplissage ou matériau de comblement incorpore des poches de fluides qui deviennent autant de zones de faiblesse mécanique après durcissement et d'éventuels foyers d'infection provoquant des scellements incomplets. Les conséquences d'un scellement incomplet, irrégulier sont bien documentées dans la littérature. Voir par exemple: Prothèse totale de hanche de Charnley, International Symposium - Lyon 1995, édité par Groupe A.C.O.R.A. En résumé, ces conséquences sont: Fissuration, prise de jeu de l'implant dans le manteau formé par un composé de remplissage avec abrasion due aux micro-mouvements, prise de jeu entre le ciment et l'os, avec lyse et résorption de l'os hôte et descellement entraînant une reprise difficile.

Le propos de l'invention est d'éradiquer ou tout au moins réduire le plus possible ces causes de descellement en travaillant sur la géométrie et la surface de l'implant pour évacuer les fluides perturbateurs et autres débris durant la phase de faible viscosité et pâteuse du composé de remplissage.

Il est un but de la présente invention d'évacuer les poches d'air ou de gaz incluses dans le composé de remplissage lors de son mélange et pendant la réaction de prise du composé de remplissage, ainsi que les fluides physiologiques incorporés lors du remplissage de la cavité fémorale.

Il est un autre but de la présente invention d'équilibrer les pressions au sein du composé de remplissage avant son durcissement, en particulier dans la zone médiane et antéro-postérieure proximale de l'os, lors de la pénétration de la partie de fixation du piton dans l'os fémoral.

Un autre but encore de l'invention est de réaliser une distribution des résultantes d'appui occasionnées par le poids du corps sur la tête articulaire de manière isostatique entre l'implant et le composé de remplissage, et entre l'os et le composé de remplissage après son durcissement.

Ces buts et avantages de la présente invention sont obtenus grâce au piton de fixation osseuse selon l'invention qui est défini dans la partie caractérisante de la revendication 1.

Pour que l'invention puisse être mieux comprise, référence est faite aux dessins accompagnants dans lesquels:
- la figure 1 représente un mode de réalisation du piton de fixation osseuse pour tête articulaire fémorale selon l'invention.
- la figure 2 représente le piton de fixation osseuse de la figure 1 montrant le raccordement entre la partie d'assemblage avec la tête articulaire du piton et sa partie de fixation à l'os fémoral.
- la figure 3 est une vue arrière du piton de fixation de la figure 1.
- la figure 4 est une coupe le long de la ligne CC dans la partie de fixation du piton de la figure 1.
- la figure 5 représente un autre mode de réalisation de piton de fixation osseuse selon l'invention.
- la figure 6 est une représentation de l'extrémité des pitons de fixation des figures 1 et 5 montrant une pièce utilisée pour orienter le piton pendant sa descente dans l'os fémoral.

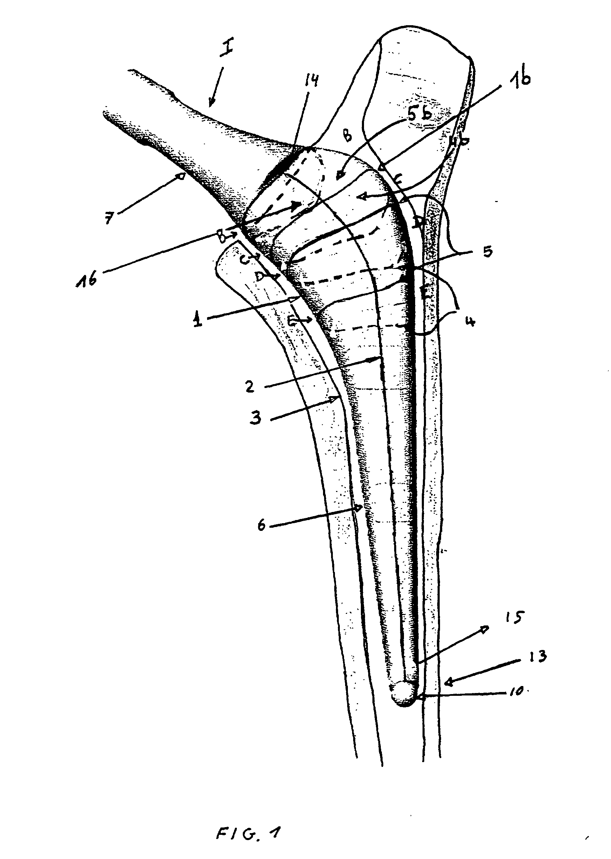

La figure 1 représente un mode de réalisation du piton de fixation osseuse I selon l'invention pour tête articulaire fémorale comprenant une partie d'assemblage 7 avec la tête articulaire fémorale, cette partie d'assemblage comportant un effet de coincement dans cette tête articulaire fémorale, cet effet de coincement variant selon le type de tête articulaire choisie. Le piton de fixation osseuse I comporte également une partie de fixation 6 destinée à être fixée à l'os fémoral 3 dans le tube osseux 13 par l'intermédiaire d'un composé introduit à l'état pâteux dans le tube osseux et remplissant l'espace entre l'os et le piton de fixation I. Après durcissement de ce composé de remplissage, le piton de fixation I est ancré dans l'os fémoral.

La partie de fixation 6 du piton comporte un bord médian 1 qui suit une courbe concave, dite courbe guide 2 dont le rayon est en harmonie avec la forme osseuse fémorale 3 correspondante.

La partie de fixation 6 et la partie d'assemblage 7 sont raccordées ensemble par l'intermédiaire d'un rebord 14.

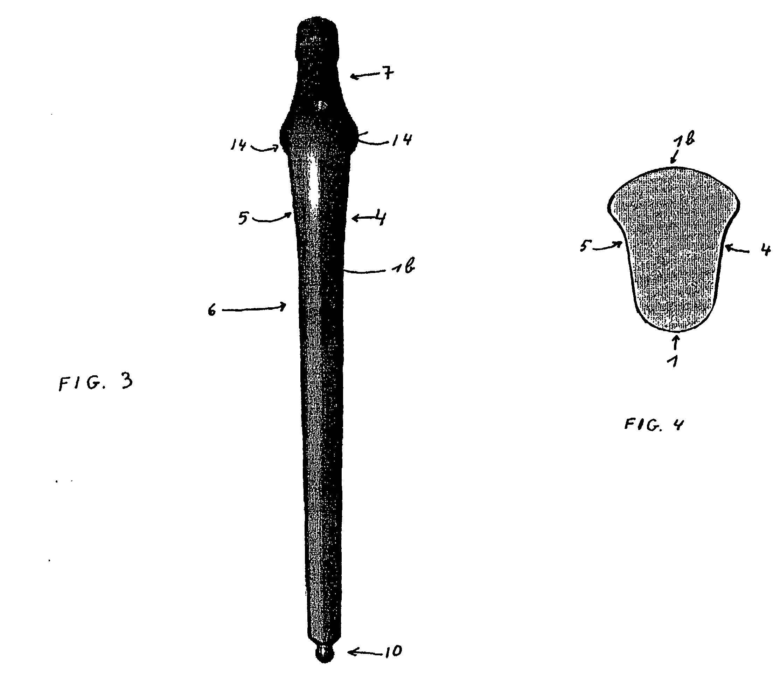

Pour atteindre les buts de l'invention, la partie de fixation 6 a une forme en "étrave de navire" selon laquelle cette partie de fixation 6 s'évase depuis son extrémité distale 15 jusqu'au rebord 14 comme on peut le voir dans la figure 3 et simultanément, cette partie de fixation 6 s'évase également dans sa partie proximale 16 depuis son bord médian 1 jusqu'au bord opposé 1b, comme on peut le voir dans la figure 4, créant ainsi des évasements arrière 4 et avant 5. Ces évasements sont montrés dans la figure 1 par les différentes sections BB à EE ainsi que dans la figure 4. Cette forme en "étrave du navire" avec l'évasement vers le haut et cette formation des évasements arrière 4 et avant 5 créent une ligne de fuite optimale des fluides et déchets à évacuer latéralement et vers le haut. D'autre part, cette forme en étrave ainsi que les évasements arrière 4 et avant 5 constituent des zones de mise en pression isostatique 4b et 5b de la pâte de remplissage qui équilibrent les pressions au sein du composé de remplissage avant son durcissement, en particulier dans la zone médiane et antéro-postérieure proximale de l'os, lors de la pénétration de la partie de fixation dont la forme répartit de façon symétrique et équilibre la masse du composé de remplissage ou d'ancrage.

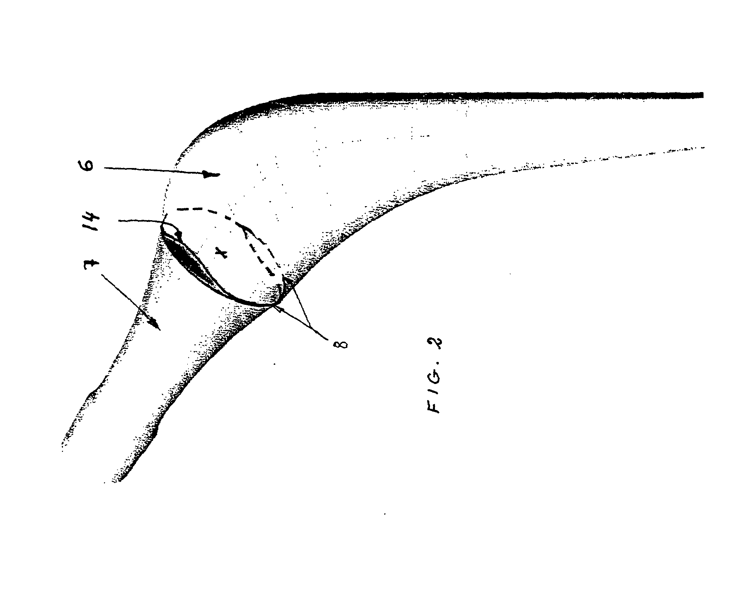

La figure 2 montre plus particulièrement le rebord 14 raccordant la partie d'assemblage 7 et la partie de fixation 6. Selon un mode de réalisation préféré de l'invention, ce rebord est oblique par rapport à la partie de fixation 6 et se caractérise par une absence de rebord médian, (c.-à-d. par une absence de rebord adjacent au bord médian 1) et une absence de rebord adjacent au bord opposé 1b. Cette structure est obtenue en donnant à la partie d'assemblage 7 et à la partie de fixation 6, à l'endroit de leur raccordement des rayons égaux et isocentriques. Ceci crée un angle de fuite externe vers la base du grand trochanter du fémur. Cet angle de fuite externe facilite encore davantage l'évacuation des fluides ou de l'excédent de pâte de remplissage.

La partie de fixation 6 du piton I possède, de préférence, une surface glacée destinée à faciliter, lors de sa pénétration, avant durcissement, l'écoulement des fluides et du composé de remplissage, pour supprimer l'abrasion due aux micro-mouvements par l'effet ventouse obtenu grâce à ladite surface glacée et permettant un contact permanent même en cas de décollement accidentel dû à une surcharge entre la partie de fixation 6 et le composé de remplissage solidifié.

A l'extrémité distale 15 de la partie de fixation 6, opposée à la tête articulaire, la partie de fixation comprend un élément de guidage 10 qui est destiné à éviter le coincement du piton lors de son introduction dans la pâte de comblement remplissant le tube osseux 13. Il doit cependant être bien compris qu'il est également dans les limites de la présent invention de réaliser des pitons de fixation osseuse sans cet élément de guidage si on le désire.

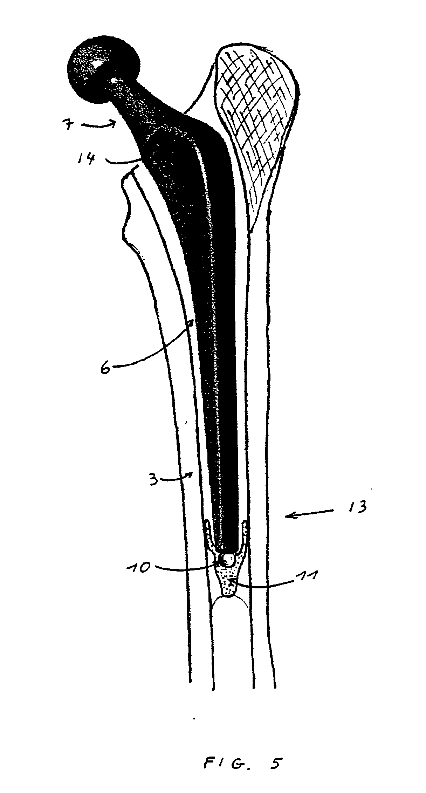

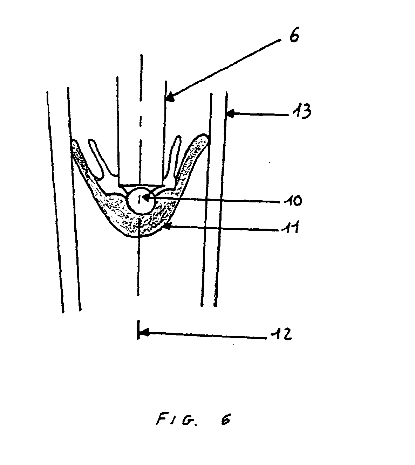

Selon un autre mode de réalisation, l'élément de guidage 10 peut recevoir une pièce 11 (voir figure 6) qui enveloppe au moins partiellement l'élément de guidage 10, dont le but est d'orienter le piton pendant sa descente le long de la fibre neutre 12 du tube osseux 13.Sa position dans le tube osseux est également montrée dans la figure 5. Cette pièce est connue dans la technique et ne nécessite pas une description supplémentaire.

La figure 5 représente un autre mode de réalisation de piton de fixation osseuse selon l'invention ayant toutes les caractéristiques nécessaires pour atteindre les buts de l'invention, décrites en rapport avec le mode de réalisation de la figure 1, mais dont la partie d'assemblage 7 fait partie intégrante avec la tête articulaire fémorale de sorte que l'ensemble partie de fixation 6, partie d'assemblage 7 et tête articulaire ne forment qu'une seule pièce.

L'invention peut être mise en oeuvre dans d'autres formes spécifiques. Les formes de réalisation de la présente invention sont par conséquent considérées dans tous leurs aspects comme étant données à titre d'illustration et non de restriction, le cadre de l'invention étant indiqué par les revendications annexées plutôt que par le description ci-dessus.

## Revendications

1. Piton de fixation osseuse (I) pour tête articulaire fémorale comprenant une partie d'assemblage (7) avec ladite tête articulaire fémorale, et une partie de fixation (6) destinée à être fixée à l'os fémoral (3) dans le tube osseux (13), ladite partie d'assemblage (7) comportant un effet de coincement dans cette tête articulaire fémorale, la partie de fixation (6) étant destinée à être ancrée dans le tube osseux par l'intermédiaire d'un composé de remplissage ou ciment remplissant l'espace entre l'os et le piton de fixation (I), la partie de fixation (6) ayant un bord médian (1) qui suit une courbe guide (2) du piton (I), dont le rayon est en harmonie avec la forme osseuse de fémur, la partie de fixation (6) et la partie d'assemblage (7) étant raccordées par l'intermédiaire d'un rebord (14), **caractérisé en ce que** la partie de fixation (6) a une forme en "étrave de navire" en s'évasant depuis son extrémité distale (15) jusqu'au rebord (14) et en s'évasant simultanément dans sa partie proximale (16) depuis son bord médian (1) jusqu'à un bord opposé (1b), créant ainsi des évasements arrière (4) et avant (5).

2. Piton de fixation osseuse (I) selon la revendication 1 **caractérisé en ce que** la forme "en étrave de navire" de la partie de fixation (6) crée une ligne de fuite optimale des poches d'air, de gaz, et de déchets présents dans le composé de remplissage lors de son mélange et pendant sa réaction de prise dans le tube osseux (13) et cette forme équilibre également les pressions au sein du composé de remplissage avant son durcissement et répartit de façon symétrique et équilibre la masse du composé de remplissage.

3. Piton de fixation osseuse (I) selon la revendication 1 **caractérisé en ce que** la partie de fixation (6) du piton (I) possède, de préférence, une surface glacée destinée à faciliter, lors de sa pénétration, avant durcissement, l'écoulement des fluides et du composé de remplissage, pour supprimer l'abrasion due aux micro-mouvements par l'effet ventouse obtenu grâce à ladite surface glacée et permettant un contact permanent même en cas de décollement accidentel dû a une surcharge entre la partie de fixation (6) et le composé de remplissage solidifié.

4. Piton de fixation osseuse (I) selon l'une quelconque des revendications 1 à 3 **caractérisé en ce que** le rebord (14) est oblique par rapport à la partie de fixation (6) et il se **caractérise par** une absence de rebord médian, c.-à-d. une absence de rebord adjacent au bord médian (1) du piton et une absence de rebord adjacent au bord (1b) opposé au bord médian.

5. Piton de fixation osseuse (I) selon la revendication 4 **caractérisé en ce que** le rebord (14) forme un angle de fuite externe favorisant l'évacuation des poches d'air, de gaz, de déchets et de l'excès de pâte de remplissage vers la base du grand trochanter du fémur.

6. Piton de fixation osseuse (I) selon la revendication 1 **caractérisé en ce que** la forme en "étrave de navire" de la partie de fixation (6) réalise un calage automatique du piton, même en cas de perte d'adhérence secondaire du composé ou lors de son refroidissement après la réaction exothermique de durcissement du composé de remplissage.

7. Piton de fixation osseuse (I) selon l'une quelconque des revendications 1 à 6 **caractérisé en ce que** la partie de fixation (6) comporte à son extrémité distale un élément de guidage (10) destiné à éviter le coincement du piton ou la perforation osseuse pouvant en résulter, lors de son introduction dans la pâte de remplissage.

8. Piton de fixation osseuse (I) selon la revendication 7 **caractérisé en ce que** l'élément de guidage (10) est en mousse et a une forme sphérique, olivaire ou cylindro-sphérique.

9. Piton de fixation osseuse selon l'une quelconque des revendications 7 ou 8 **caractérisé en ce que** l'élément de guidage (10) peut recevoir une pièce (11) l'enveloppant, même partiellement et destinée à orienter la descente du piton dans la pâte de remplissage le long de la fibre neutre (12) du tube osseux.

10. Piton de fixation osseuse (I) selon la revendication 1 **caractérisé en ce que** la partie d'assemblage (7) fait partie intégrante avec la tête articulaire fémorale de sorte que l'ensemble partie d'assemblage (7), la partie de fixation (6) et la tête articulaire fémorale ne forment qu'une seule pièce.

## Patentansprüche

1. Schraube zur Knochenfixierung (I) für einen Oberschenkelgelenkskopf, umfassend einen Teil (7) zur Zusammenfügung mit dem Oberschenkelgelenkskopf und einen Fixierungsteil (6), der dazu bestimmt ist, am Oberschenkelknochen (3) in der Knochenröhre (13) fixiert zu werden, wobei der Zusammenfügungsteil (7) eine Klemmwirkung in diesem Oberschenkelgelenkskopf umfasst, wobei der Fixierungsteil (6) dazu bestimmt ist, in der Knochenröhre mit Hilfe einer Füllverbindung oder eines Zements, die den Raum zwischen dem Knochen und der Fixierungsschraube (I) ausfüllen, verankert zu werden, wobei der Fixierungsteil (6) einen mittleren Rand (1) aufweist, der einer Führungskurve (2) der Schraube (I) folgt, deren Radius mit der Form des Oberschenkelknochens im Einklang steht, wobei der Fixierungsteil (6) und der Zusammenfügungsteil (7) mit Hilfe einer Leiste (14) verbunden sind, **dadurch gekennzeichnet, dass** der Fixierungsteil (6) die Form eines "Schiffbugs" aufweist, indem er sich von seinem distalen Ende (15) bis zur Leiste (14) erweitert und indem er sich gleichzeitig in seinem proximalen Teil (16) von seinem mittleren Rand (1) bis zu einem gegenüber liegenden Rand (1b) erweitert und auf diese Weise hintere (4) und vordere (5) Erweiterungen bildet.

2. Schraube (I) zur Knochenfixierung nach Anspruch 1, **dadurch gekennzeichnet, dass** die Form eines "Schiffbugs" des Fixierungsteils (6) eine optimale Ableitlinie für die Luft-, Gastaschen und Abfälle, die in der Füllverbindung bei ihrer Mischung und Festwerdereaktion in der Knochenröhre (13) vorhanden sind, erzeugt, und dass diese Form auch die Drucke innerhalb der Füllverbindung vor ihrem Härten ausgleicht und die Masse der Füllverbindung symmetrisch verteilt und ausgleicht.

3. Schraube (I) zur Knochenfixierung nach Anspruch 1, **dadurch gekennzeichnet, dass** der Fixierungsteil (6) der Schraube (I) vorzugsweise eine geglättete Oberfläche umfasst, die dazu bestimmt ist, bei seinem Eindringen vor dem Härten das Abfließen der Fluide und der Füllverbindung zu erleichtern, um das Abschleifen auf Grund der Mikrobewegungen durch Saugeffekt zu verhindern, der dank der geglätteten Oberfläche erzielt wird, wobei ein ständiger Kontakt, auch im Falle eines zufälligen Loslösens auf Grund einer Überlastung zwischen dem Fixierungsteil (6) und der verfestigten Füllverbindung ermöglicht wird.

4. Schraube (I) zur Knochenfixierung nach einem der Ansprüche 1 bis 3, **dadurch gekennzeichnet, dass** die Leiste (14) in bezug auf den Fixierungsteil (6) schräg ist und durch ein Fehlen einer mittleren Leiste, d.h. ein Fehlen einer an den mittleren Rand (1) der Schraube angrenzenden Leiste, und ein Fehlen einer an den dem mittleren Rand gegenüber liegenden Rand (1b) angrenzenden Leiste gekennzeichnet ist.

5. Schraube (I) zur Knochenfixierung nach Anspruch 4, **dadurch gekennzeichnet, dass** die Leiste (14) einen äußeren Abflusswinkel bildet, der die Beseitigung der Luft-, Gastaschen, Abfälle und Überschüsse von Füllpaste zu der Basis des großen Trochanters des Oberschenkelknochens begünstigt.

6. Schraube (I) zur Knochenfixierung nach Anspruch 1, **dadurch gekennzeichnet, dass** die Form eines "Schiffbugs" des Fixierungsteils (6) eine automatische Verkeilung der Schraube auch im Falle eines sekundären Haftverlustes der Verbindung oder bei ihrer Abkühlung nach der exothermen Härtungsreaktion der Füllverbindung durchführt.

7. Schraube (I) zur Knochenfixierung nach einem der Ansprüche 1 bis 6, **dadurch gekennzeichnet, dass** der Fixierungsteil (6) an seinem distalen Ende ein Führungselement (10) umfasst, das dazu bestimmt ist, das Einklemmen der Schraube oder die Knochenperforation zu vermeiden, die sich bei ihrer Einführung in die Füllpaste ergeben können.

8. Schraube (I) zur Knochenfixierung nach Anspruch 7, **dadurch gekennzeichnet, dass** das Führungselement (10) aus Schaumstoff ist und eine kugelförmige, olivenförmige oder zylindrisch-kugelförmige Form hat.

9. Schraube zur Knochenfixierung nach einem der Ansprüche 7 oder 8, **dadurch gekennzeichnet, dass** das Führungselement (10) ein Teil (11), das es auch nur teilweise umhüllt, aufnehmen kann, das dazu bestimmt ist, das Absenken der Schraube in die Füllpaste entlang der neutralen Faser (12) der Knochenröhre auszurichten.

10. Schraube (I) zur Knochenfixierung nach Anspruch 1, **dadurch gekennzeichnet, dass** der Zusammenfügungsteil (7) integrierender Bestandteil des Oberschenkelgelenkskopfes ist, so dass die Einheit Zusammenfügungsteil (7), Fixierungsteil (6) und Oberschenkelgelenkskopf nur ein Stück bilden.

## Claims

1. A bone fixation pin (I) for the articular femoral head comprising a part (7) for assembly with the said articular femoral head, and a fixation part (6) designed to be fixed to the femoral bone (3) in the bone canal (13), the said assembly part (7) comprising a binding effect in this articular femoral head, the fixation part (6) being designed to be anchored in the bone canal through a filling compound or cement filling the space between the bone and the fixation pin (I), the fixation part (6) having a median edge (1) which follows a curve guide (2) of the pin (I), wherein the radius is in conformity with the bone shape of the femur, the fixation part (6) and the assembly part (7) being connected through a flange (14), **characterized in that** the fixation part (6) has a "ship bow" shape by flaring from its distal end (15) to the flange (14) and by flaring simultaneously in its proximal part (16) from its median edge (1) to the opposite edge (1b), thus creating rear (4) and front (5) flares.

2. The bone fixation pin (I) according to claim 1 **characterized in that** the "ship bow" shape of the fixation part (6) creates an optimal leaking path for pockets of air, gas and waste present in the filling compound during its mixing and during its setting reaction in the bone canal (13) and this shape also balances the pressures within the filling compound before it hardens and symmetrically distributes and equalizes the mass of the filling compound.

3. The bone fixation pin (I) according to claim 1 **characterized in that** the fixation part (6) of the pin (I) has, preferably, a smoothed surface designed to facilitate, during its penetration and before it hardens, the flow of fluids and filling compound, to ease abrasion due to micro-movements by the suction effect obtained because of the said smoothed surface and allowing a permanent contact even in case of accidental detachment due to an overload between the fixation part (6) and the solidified filling compound.

4. The bone fixation pin (I) according to any one of claims 1 to 3 **characterized in that** the flange (14) is oblique compared to the fixation part (6) and is **characterized by** an absence of a median flange, that is, by an absence of a flange adjacent to the median edge (1) of the pin and an absence of a flange adjacent to the edge (1b) opposite from the median edge.

5. The bone fixation pin (I) according to claim 4 **characterized in that** the flange (14) forms an external departure angle which facilitates the evacuation of air pockets, gas, wastes and excess filling compound towards the base of the greater trochanter of the femur.

6. The bone fixation pin (I) according to claim 1 **characterized in that** the "ship's bow" shape of the fixation part (6) performs an automatic adjustment of the pin, even in the case of secondary loss of adhesion of the compound or during its cooling after the exothermic reaction of the hardening of the filling compound.

7. The bone fixation pin (I) according to any one of claims 1 to 6 **characterized in that** the fixation part (6) comprises a guiding element (10) at its distal end designed to avoid jamming of the pin or bone perforation that may occur during introduction into the filling compound.

8. The bone fixation pin (I) according to claim 7 **characterized in that** the guiding element (10) is in foam and has a spherical, oval or cylindro-spherical shape.

9. The bone fixation pin according to any one of claims 7 or 8 **characterized in that** the guiding element (10) may receive an enveloping piece (11), even partially, designed to orient the descent of the pin into the filling compound along the neutral axis (12) of the bone canal.

10. The bone fixation pin (I) according to claim 1 **characterized in that** the assembly part (7) makes an integral part with the articular femoral head so that the assembly part (7), fixation part (6), and articular femoral head assembly forms only a single piece.
